(19) Europäisches Patentamt
European Patent Office
Office européen des brevets

(11) Publication number: **0 313 202 A2**

(12) **EUROPEAN PATENT APPLICATION**

(21) Application number: **88308358.6**

(22) Date of filing: **09.09.88**

(51) Int. Cl.[4]: **C07D 277/64 , C07D 513/04 , C07D 277/60 , //(C07D513/04, 277:00,213:00)**

(30) Priority: **16.09.87 US 97033**

(43) Date of publication of application: **26.04.89 Bulletin 89/17**

(84) Designated Contracting States: **AT BE CH DE ES FR GB GR IT LI LU NL SE**

(71) Applicant: **PFIZER INC.**
**235 East 42nd Street**
**New York, N.Y. 10017(US)**

(72) Inventor: **Mylari, Banavara Lakshmana**
**6 Quinley Way**
**Waterford Connecticut(US)**
Inventor: **Zembrowski, William James**
**1315 Route 163**
**Oakdale Connecticut(US)**

(74) Representative: **Wood, David John et al**
**Pfizer Limited Ramsgate Road**
**Sandwich Kent CT13 9NJ(GB)**

(54) **Process for preparing halomethyl thiazoles.**

(57) A process for the preparation of a compound of the formula

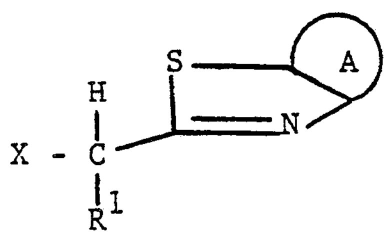

I

wherein $R^1$ is hydrogen or $(C_1\text{-}C_4)$alkyl, X is chloro or bromo and A represents a 6 membered carbocylic aromatic or a 5 or 6 membered heterocyclic ring which is optionally substituted by one of iodo or trifluoromethylthio, or one or two of fluoro, chloro, bromo, $(C_1\text{-}C_4)$alkyl, $(C_1\text{-}C_4)$alkoxy, $(C_1\text{-}C_4)$alkylthio, $(C_1\text{-}C_4)$alkylsulfinyl, $(C_1\text{-}C_4)$alkylsulfonyl, or trifluoromethyl, comprising reacting haloacetonitrile with a compound of the formula

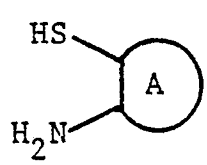

II ..

EP 0 313 202 A2

wherein A is as defined above or a hydrohalide salt thereof.

The compounds of the formula (I) are useful intermediates in the preparation of heterocyclic oxophthalazinyl acetic acids having aldose reductase inhibitory activity. Some of the compounds of the formula (I) are novel, and these also form a part of the invention.

## PROCESS FOR PREPARING HALOALKYL THIAZOLES AND HALOALKYL THIAZOLES PREPARED THERBY

The present invention relates to processes and novel intermediates for the preparation of halomethyl thiazoles. Halomethyl thiazoles are useful intermediates in the preparation of heterocyclic oxophthalazinyl acetic acids having aldose reductase inhibitory activity. Such compounds are disclosed in European Patent Application Publication Number 0222576. Halomethyl thiazoles are referred to in United States Patents 4,075,207, 4,554,355 and 4,550,172.

The prior art methods for preparing 2-halomethyl thiazoles suffer from major disadvantages. In a well known method, a 2-methyl benzothiazole is brominated in a standard procedure with N-bromosuccinimide to obtain only around 30% of the desired 2-bromomethyl benzothiazoles upon difficult separation from unreacted starting material and over-brominated by-products. In a second procedure (Synthesis, 102-103 (1979), o-aminobenzene thiols are condensed with methyl chloroacetimidate, which in turn is prepared from chloroacetonitrile. In a third method (see United States Patents 4,723,010 and 4,748,280), an appropriate o-aminobenzene thiol is condensed with 2-chloro-1,1,1-trialkoxy ethane, which is prepared in one or two steps starting from chloroacetonitrile. The second and third methods are limited to the preparation of 2-chloromethylbenzothiazoles, i.e. they are not suitable for the preparation of 2-bromomethylbenzothiazoles. A fourth method comprises reacting an appropriate o-aminobenzenethiol with $ClCH_2COCl$ (Japanese Patent Publication (Kokai) 77 66531). This method provides very poor yields.

None of the prior art procedures is suitable for the direct preparation of 2-alkyl branched halomethyl thiazoles. In the literature (J. Ind. Chem. Soc., 566 (1979)), alkyl branched compounds are made by a two-step procedure by interaction of o-aminobenzene thiol with lactic acid to obtain 2-alkyl branched hydroxymethyl benzothiazole and its subsequent conversion to the desired compound in a standard reaction with thionyl chloride.

The process of the present invention is advantageous because, compared to prior art processes, it provides for preparing (a) 2-halomethylthiazoles by reacting 2-aminothiols directly with haloacetonitriles, while prior art procedures are protracted in that they require derivatives of haloacetonitriles; and (b) 2-alkyl-2-halomethylthiazoles in one step. In addition, the process of the present invention enables the preparation of both 2-chloro and 2-bromomethylthiazoles while the prior art processes are restricted to the preparation of 2-chloromethylthiazoles. The 2-bromomethyl derivatives are preferred in some nucleophilic displacement reactions because they are more reactive than the 2-chloromethyl analogues.

The present invention relates to a process for the preparation of a compound of the formula

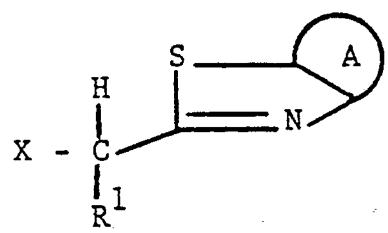

I

wherein $R^1$ is hydrogen or $(C_1-C_4)$alkyl, X is chloro or bromo, and A represents a 6 membered carbocyclic aromatic or a 5 or 6 membered heterocyclic ring which is optionally subsituted by one of iodo or trifluoromethylthio, or one or two of fluoro, chloro, bromo, $(C_1-C_4)$alkyl, $(C_1-C_4)$alkoxy, $(C_1-C_4)$alkylthio, $(C_1-C_4)$alkylsulfinyl, $(C_1-C_4)$alkylsulfonyl, or trifluoromethyl, comprising reacting haloacetonitrile with a compound of the formula

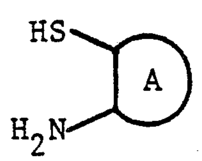

II

wherein A is as defined above or a hydrohalide salt thereof.

When A is a 6 membered carbocyclic aromatic ring, it may be viewed as a benzene ring to which a sulfur atom and a nitrogen atom, in the ortho position with respect to each other, are attached. If such a ring

has no other substituents, it may also be referred to herein as an unsubstituted benzene ring.

Preferred heterocyclic rings are 5 membered aromatic rings having a single oxygen or sulfur atom in the ring and 6 membered aromatic rings with one or two nitrogen atoms in the ring. Specific examples of suitable heterocyclic rings are the following:

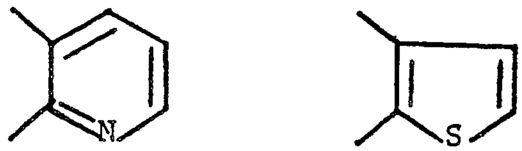

The foregoing two rings may be viewed, respectively, as pyridine and thiophene rings to which nitrogen and sulfur are attached. If there are no other substituents, they will be referred to as unsubstituted pyridine and thiophene rings.

In view of the foregoing, it will be clear that specific examples of suitable compounds of the formula II are the following:

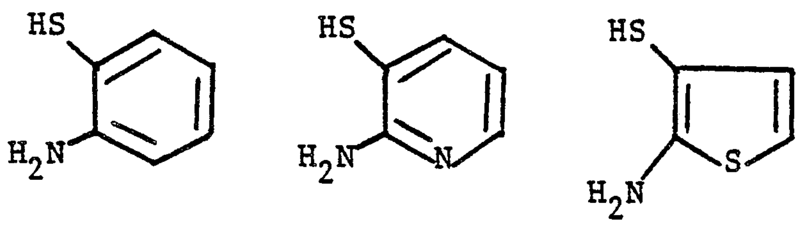

The compound of the formula II is preferably used in the form of its hydrohalide salt (e.g., the hydrochloride, hydrobromide or hydroiodide salt) and is most preferably used as the hydrochloride salt. The hydrochloride salt may be added to the reaction medium or formed in situ.

The present invention also relates to compounds of the formula I wherein $R^1$ is hydrogen or ($C_1$-$C_4$)-alkyl, X is chloro or bromo, and when X is bromo, A represents a ring selected from the group consisting of benzene, pyridine and thiophene, each of said benzene, pyridine and thiophene rings being optionally substituted by one of iodo or trifluoromethylthio, two of fluoro, or one or two of chloro, bromo, ($C_1$-$C_4$)-alkylthio, ($C_1$-$C_4$)alkylsulfinyl, ($C_1$-$C_4$)alkylsulfonyl, or trifluoromethyl; and when X is chloro and $R^1$ is as defined above, A represents a thiophene ring optionally subsituted as described above; and when X is chloro and $R^1$ is ($C_1$-$C_4$)alkyl, A also represents a pyridine ring optionally subsituted as described above, or a benzene ring optionally subsituted by one trifluoromethylthio, or one or two of ($C_1$-$C_4$)alkylthio, ($C_1$-$C_4$)-alkylsulfinyl, ($C_1$-$C_4$)alkylsulfonyl, or trifluoromethyl, or two of fluoro, chloro, bromo, ($C_1$-$C_4$)alkyl or ($C_1$-$C_4$)-alkoxy.

In a preferred embodiment, A is a benzene ring substituted as described above. More preferred compounds of the present invention are compounds of the formula I wherein A is a benzene ring and wherein the resulting benzothiazole is substituted at the 5-position with $CF_3$; at the 4- and 5-positions or the 5- and 7- positions with F; or at the 4- and 5-positions or the 5- and 7- positions with Cl; or at the 4- and 7-positions with Cl or F. Specific preferred compounds are the following:

2-bromomethyl-5-trifluoromethylbenzothiazole;
2-bromomethyl-4,5-difluorobenzothiazole;
2-bromomethyl-4,5-dichlorobenzothiazole;
2-bromomethyl-5,7-dichlorobenzothiazole; and
2-bromomethyl-5,7-difluorobenzothiazole.

## Detailed Description of the Invention

The process for preparation of compounds of the formula I is conducted neat or in the presence of a alcoholic solvent. Suitable solvents include ($C_1$-$C_4$)alkanols (e.g., ethanol). The haloacetonitrile may be chloroacetonitrile or bromoacetonitrile. The reaction temperature should be in the range of about 60 to about

140°C, preferably about 60 to about 80°C. Generally, the reaction will be conducted at the reflux temperature of the reaction mixture. The reaction pressure is not critical. Generally, the reaction will be conducted at a pressure of about 1 to about 2 atmospheres, preferably at ambient pressure.

The compounds prepared by the process of the present invention may be reacted with oxophthalizinyl acetic acids, or their esters, to prepare heterocyclic oxophthalizinyl acetic acids having aldose reductase inhibitory activity. For example, a halomethyl benzothiazole prepared by the process of the present invention may be reacted with oxophthalazinyl acetic acid ester as follows:

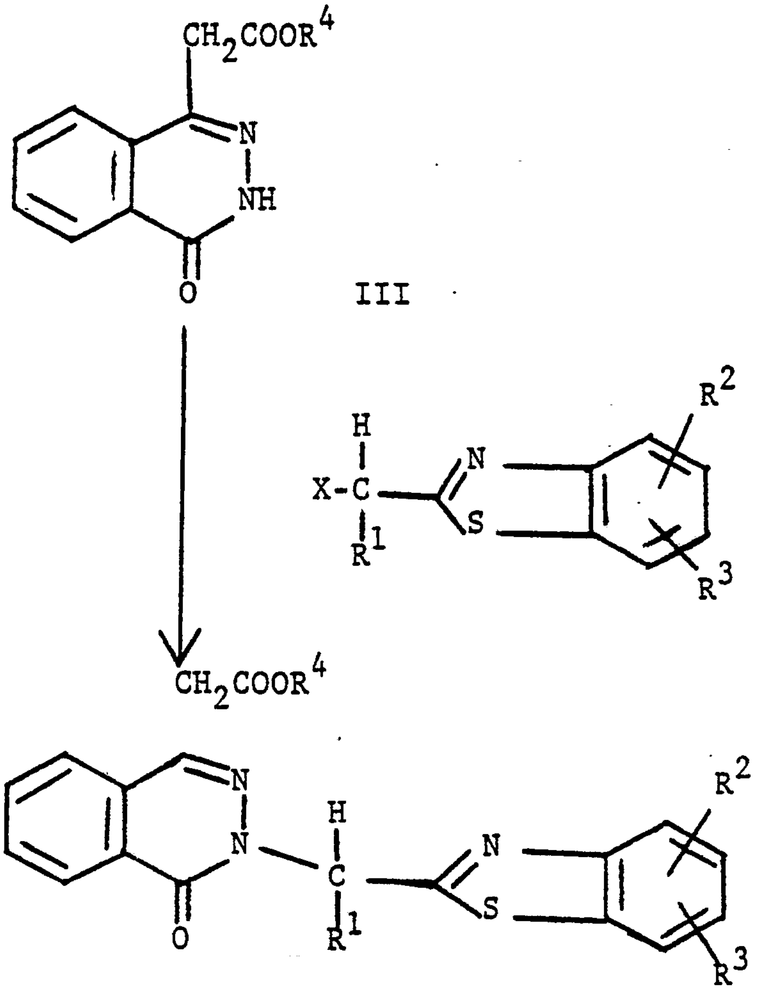

III

IV

In the above reaction scheme, $R^4$ is $(C_1-C_4)$alkyl, $R^1$ and X are as defined above, and $R^2$ and $R^3$ represent the substituents on ring A described above.

When reacting compounds III wherein $R^4$ is alkyl (e.g., methyl or ethyl), the reaction is generally carried out in a polar solvent such as an alkanol having 1 to 4 carbon atoms, e.g. methanol or ethanol, dioxan, dimethylformamide, or dimethylsulfoxide, in the presence of a base. Suitable bases are alkali metal hydride or alkoxide of 1 to 4 carbon atoms, such as sodium or potassium hydride, methoxide or ethoxide. When a hydride is used, a non-aqueous solvent such as dimethylformamide is required. When reacting compounds III wherein $R^5$ is hydrogen, obtained on hydrolysis of compounds (IV) wherein $R^5$ is alkyl, it is necessary for at least two molar equivalents of the base to be present, since the first molar equivalent reacts with the carboxylic acid radical of such a compound. In addition, when reacting such compounds, it is preferable to use a hydroxylic solvent to minimize production of the corresponding ester. The reaction to form compound IV may be at room temperatue, or at higher temperatures to accelerate the process. The compounds of formula IV wherein $R^4$ is alkyl may be hydrolyzed to obtain compounds of formula IV wherein $R^4$ is hydrogen. The hydrolysis proceeds at conventional temperatures and in the presence of acid or base such as a mineral acid, for example hydrochloric acid, or an alkali metal hydroxide or carbonate such as sodium or potassium hydroxide or carbonate. The reaction is carried out in the presence of water and a solvent, for example and alkanol of 1 to 4 carbon atoms such as methanol, or dioxane.

The compounds of formula IV and the pharmaceutically acceptable salts thereof are useful as inhibitors of the enzyme aldose reductase in the treatment of chronic complications of diabetes, such as diabetic cataracts, retinopathy and neuropathy. Such treatment includes both the prevention and alleviation of such conditions. The compounds of the formula IV or pharmaceutically acceptable salts thereof may be administered alone or in combination with pharmaceutically acceptable carriers, in either single or multiple doses. The compounds or pharmaceutically acceptable salts thereof may be administered to a subject in need of treatment by a variety of conventional routes of administration, including orally, parenterally and topically. In general, these compounds will be administered orally or parenterally at dosages between about 0.5 and 25 mg/kg body weight of the subject to be treated per day, preferably from about 1.0 to 10 mg/kg. However, some variation in dosage will necessarily occur depending on the condition of the subject being treated.

Compounds of formula IV or their pharmaceutically acceptable salts may also be advantageously employed for the preparation of aqueous pharmaceutical compositions for suitable for use as ophthalmic solutions. Such ophthalmic solutions are of principal interest for the treatment of diabetic cataracts by topical administration. For such treatment, the compounds are administered to the eye in an ophthalmic preparation containing a compound of formula IV or a pharmaceutically acceptable salt thereof in a concentration from about 0.01 to about 1% by weight, preferably from about 0.05 to about 0.5% in a pharmaceutically acceptable solution, suspension or ointment. Such preparations will generally be administered to the eye in the form of drops or by bathing the eye in the preparation.

The following non-limiting Examples illustrate the present invention. All melting points referred to in the Examples are uncorrected.

## Example 1

### 2-Chloromethylbenzothiazole

A mixture of 2-aminobenzenethiol hydrochloride (3.23 g), chloroacetonitrile (1.51 g) and ethanol (20 ml) was refluxed for 4 hours. The residue obtained upon evaporation of ethanol was extracted with ethylacetate and the extract was washed first with 10% aqueous HCl (2 x 10 ml) and then with water (2 x 20 ml). The organic layer was dried and evaporated to obtain a crude solid, which was purified by flash chromatography on silica gel with methylene chloride as the solvent to obtain the title compound (yield: 3.15 g). Its m.p. 34°C was identical with that reported in the literature (Synthesis, 102-103 (1979)).

## Example 2

### 2-Bromomethylbenzothiazole

A mixture of 2-aminobenzenethiol hydrochloride (3.23 g), bromoacetonitrile (2.4 g) and ethanol (20 ml) was refluxed for 3 hours. Upon work-up of the reaction following the procedure of Example 1, the title compound was obtained.

## Example 3

### 2- Chloromethylthiazolo[5,4-b]pyridine

A mixture of 3-amino-2-mercaptopyridine hydrochloride (312 mg) (J. Org. Chem., 2652, (1963)),

chloroacetonitrile (189 mg) and ethanol (5 ml) was refluxed for 6 hours. The ethanol was evaporated and the residue stirred with water, filtered and air dried to yield the title compound (yield, 200 mg), m.p. 71-73° C.

Example 4

2-(alpha-Chloroethyl)-5-trifluoromethyl-benzothiazole

A mixture of 3-amino-4-metcaptobenzotrifluoride hydrochloride (2.29 g), 2-chloropropionitrile (1.07 g) and ethanol (20 ml) was refluxed overnight. Upon evaporation of ethanol, the residue was extracted with methylene chloride. The extract was washed with water (2 x 10 ml), dried over anhydrous magnesium sulfate and evaporated to obtain a clear oil (yield: 2.23 g); $^{1}$HNMR($CDCl_3$, 60 MHz), 2.1 (d, J = 6 Hz, 3H), 5.4 (q, J = 6 Hz, 1H), 7.5-8.3 (m, 3H).

**Claims**

1. A process for the preparation of a compound of the formula

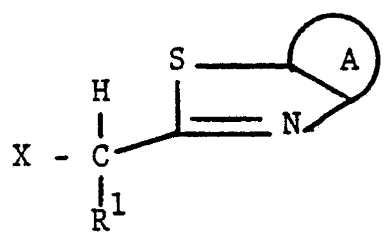

I

wherein $R^1$ is hydrogen or ($C_1$-$C_4$)alkyl, X is chloro or bromo, and A represents a 6 membered carbocyclic aromatic or 5 or 6 membered heterocyclic ring which is optionally substituted by one of iodo or trifluoromethylthio, or one or two of fluoro, chloro, bromo, ($C_1$-$C_4$)alkyl, ($C_1$-$C_4$)alkoxy, ($C_1$-$C_4$)alkylthio, ($C_1$-$C_4$)alkylsulfinyl, ($C_1$-$C_4$)alkylsulfonyl, or trifluoromethyl, comprising reacting haloacetonitrile with a compound of the formula

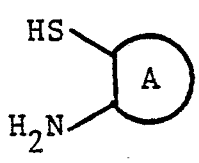

II

wherein A is as defined above or a hydrohalide salt thereof.

2. A process according to claim 1, wherein said compound of the formula II is present as its hydrohalide salt.

3. A process according to claim 1 or 2 wherein A is

(a) a substituted or unsubstituted benzene ring, the substituents being defined as in claim 1;

(b) a substituted or unsubstituted 5 or 6 membered heterocyclic ring having a single oxygen or sulfur atom in the ring or a subsituted or unsubstituted 6 membered heterocyclic ring having one or two nitrogen atoms in the ring, the substituents being defined as in claim 1; or

(c) a substituted or unsubstituted pyridine or thiophene ring, the substituents being defined as in claim 1.

4. A process according to any one of claims 1 to 3, wherein the haloacetonitrile is chloroacetonitrile or bromoacetonitrile.

5. A compound of the formula

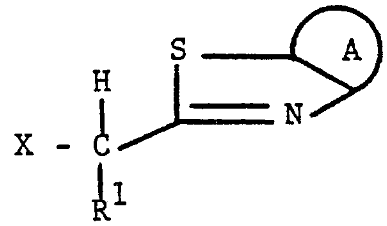

I

wherein $R^1$ is hydrogen or $(C_1-C_4)$alkyl; X is chloro or bromo; and when X is bromo, A represents a ring selected from the group consisting of benzene, pyridine and thiophene, each of said benzene, pyridine and thiophene rings being optionally substituted by one of iodo or trifluoromethylthio, two of fluoro, or one or two of chloro, bromo, $(C_1-C_4)$alkylthio, $(C_1-C_4)$alkylsulfinyl, $(C_1-C_4)$alkylsulfonyl, or trifluoromethyl; and when X is chloro and $R^1$ is as defined above, A represents a thiophene ring optionally substituted as described above; and when X is chloro and $R^1$ is $(C_1-C_4)$alkyl, A also represents a pyridine ring optionally substituted as described above, or a benzene ring optionally substituted by one trifluoromethylthio, or one or two of $(C_1-C_4)$alkylthio, $(C_1-C_4)$alkylsulfinyl, $(C_1-C_4)$alkylsulfonyl, or trifluoromethyl, or two of fluoro, chloro, bromo, $(C_1-C_4)$alkyl or $(C_1-C_4)$alkoxy.

6. A compound according to claim 5, wherein A is a benzene ring optionally substituted as described in claim 5 or a pyridine or thiophene ring, said pyridine and thiophene rings being optionally substituted as described in claim 5.

7. A compound according to claim 5, wherein A is a benzene ring and wherein the resulting benzothiazole is substituted at the 5-position with $CF_3$; at the 4-and 5-positions or the 5- and 7-positions with F; or at the 4- and 5-positions or the 5- and 7-positions with Cl.

8. A compound according to any one of claims 5 to 7, wherein X is bromo.

9. A compound according to claim 5, said compound being selected from the group consisting of
2-bromomethyl-5-trifluoromethylbenzothiazole;
2-bromomethyl-4,5-difluorobenzothiazole;
2-bromomethyl-4,5-dichlorobenzothiazole;
2-bromomethyl-5,7-diflurobenzothiazole; and
2-bromomethyl-5,7-dichlorobenzothiazole.

10. A compound according to claim 5, wherein X is chloro, $R^1$ is $(C_1-C_4)$alkyl, and A represents a benzene ring substituted by one trifluoromethylthio, or one or two of $(C_1-C_4)$alkylthio, $(C_1-C_4)$alkylsulfinyl, $(C_1-C_4)$alkylsulfonyl, or trifluoromethyl, or two of fluoro, chloro, bromo, $(C_1-C_4)$alkyl or $(C_1-C_4)$alkoxy.

Claims for the following Contracting State : ES

1. A process for the preparation of a compound of the formula

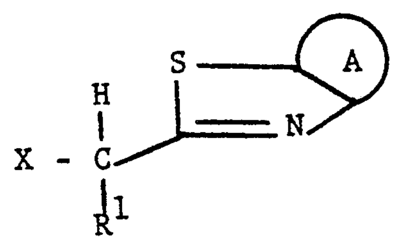

I

wherein $R^1$ is hydrogen or $(C_1-C_4)$alkyl, X is chloro or bromo, and A represents a 6 membered carbocyclic aromatic or 5 or 6 membered heterocyclic ring which is optionally substituted by one of iodo or trifluoromethylthio, or one or two of fluoro, chloro, bromo, $(C_1-C_4)$alkyl, $(C_1-C_4)$alkoxy, $(C_1-C_4)$alkylthio, $(C_1-C_4)$alkylsulfinyl, $(C_1-C_4)$alkylsulfonyl, or trifluoromethyl, comprising reacting haloacetonitrile with a compound of the formula

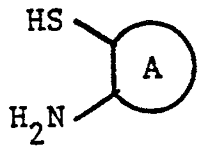

II

wherein A is as defined above or a hydrohalide salt thereof.

2. A process according to claim 1, wherein said compound of the formula II is present as its hydrohalide salt.

3. A process according to claim 1 or 2 wherein A is

(a) a substituted or unsubstituted benzene ring, the substituents being defined as in claim 1;

(b) a substituted or unsubstituted 5 or 6 membered heterocyclic ring having a single oxygen or sulfur atom in the ring or a substituted or unsubstituted 6 membered heterocyclic ring having one or two nitrogen atoms in the ring, the substituents being defined as in claim 1; or

(c) a substituted or unsubstituted pyridine or thiophene ring, the substituents being defined as in claim 1.

4. A process according to any one of claims 1 to 3, wherein the haloacetonitrile is chloroacetonitrile or bromoacetonitrile.

5. A process according to any one of claims 1 to 4 wherein $R^1$ is hydrogen or $(C_1\text{-}C_4)$alkyl; X is chloro or bromo; and when X is bromo, A represents a ring selected from the group consisting of benzene, pyridine and thiophene, each of said benzene, pyridine and thiophene rings being optionally substituted by one of iodo or trifluoromethylthio, two of fluoro, or one or two of chloro, bromo, $(C_1\text{-}C_4)$alkylthio, $(C_1\text{-}C_4)$-alkylsulfinyl, $(C_1\text{-}C_4)$alkylsulfonyl, or trifluoromethyl; and when X is chloro and $R^1$ is as defined above, A represents a thiophene ring optionally substituted as described above; and when X is chloro and $R^1$ is $(C_1\text{-}C_4)$alkyl, A also represents a pyridine ring optionally substituted as described above, or a benzene ring optionally substituted by one trifluoromethylthio, or one or two of $(C_1\text{-}C_4)$alkylthio, $(C_1\text{-}C_4)$alkylsulfinyl, $(C_1\text{-}C_4)$alkylsulfonyl, or trifluoromethyl, or two of fluoro, chloro, bromo, $(C_1\text{-}C_4)$alkyl or $(C_1\text{-}C_4)$alkoxy.

6. A process according to claim 5, wherein A is a benzene ring optionally substituted as described in claim 5 or a pyridine or thiophene ring, said pyridine and thiophene rings being optionally substituted as described in claim 5.

7. A process according to claim 5, wherein A is a benzene ring and wherein the resulting benzothiazole is substituted at the 5-position with $CF_3$; at the 4-and 5-positions or the 5- and 7-positions with F; or at the 4- and 5-positions or the 5- and 7-positions with Cl.

8. A process according to any one of claims 5 to 7, wherein X is bromo.

9. A process according to Claim 5, wherein said compound of the formula I is selected from the group consisting of

2-bromomethyl-5-trifluoromethylbenzothiazole;
2-bromomethyl-4,5-difluorobenzothiazole;
2-bromomethyl-4,5-dichlorobenzothiazole;
2-bromomethyl-5,7-difluorobenzothiazole; and
2-bromomethyl-5,7-dichlorobenzothiazole.

10. A process according to claim 5, wherein X is chloro, $R^1$ is $(C_1\text{-}C_4)$alkyl, and A represents a benzene ring substituted by one trifluoromethylthio, or one or two of $(C_1\text{-}C_4)$alkylthio, $(C_1\text{-}C_4)$alkylsulfinyl, $(C_1\text{-}C_4)$-alkylsulfonyl, or trifluoromethyl, or two of fluoro, chloro, bromo, $(C_1\text{-}C_4)$alkyl or $(C_1\text{-}C_4)$alkoxy.

11. A process according to any one of the preceding claims, characterised in that it is carried out in a $C_1\text{-}C_4$ alkanol sovent at from 60° to 140° C.